Europäisches Patentamt

European Patent Office

Office européen des brevets

(11) Veröffentlichungsnummer: **0 292 690 B1**

# EUROPÄISCHE PATENTSCHRIFT

(12)

(45) Veröffentlichungstag der Patentschrift: **25.03.92**

(21) Anmeldenummer: **88106145.1**

(22) Anmeldetag: **18.04.88**

(51) Int. Cl.⁵: **A61B 6/03**

(54) **Kühlvorrichtung für einen Computertomographen.**

(30) Priorität: **29.04.87 DE 3714311**

(43) Veröffentlichungstag der Anmeldung:
**30.11.88 Patentblatt 88/48**

(45) Bekanntmachung des Hinweises auf die
Patenterteilung:
**25.03.92 Patentblatt 92/13**

(84) Benannte Vertragsstaaten:
**DE FR**

(56) Entgegenhaltungen:
**EP-A- 0 109 206**
**EP-A- 0 182 040**
**EP-A- 0 225 964**
**DE-C- 97 510**

**PATENT ABSTRACTS OF JAPAN, Band 6, Nr.**
**125 (P-127)[1003], 10. Juli 1982, Seite 100 P**
**127 & JP-A-57-50 673**

(73) Patentinhaber: **SIEMENS AKTIENGESELL-**
**SCHAFT**
**Wittelsbacherplatz 2**
**W-8000 München 2(DE)**

(72) Erfinder: **Kröner, Hans-Jürgen**
**Ringstrasse 2**
**W-8523 Baiersdorf(DE)**

EP 0 292 690 B1

## Beschreibung

Die Erfindung betrifft einen Computertomographen mit einem feststehenden Geräteteil, in dem ein Drehkranz drehbar gelagert ist, der zu beiden Seiten einer den Patienten aufnehmenden Öffnung einen Röntgenstrahler und einen Strahlendetektor trägt, wobei der feststehende Geräteteil und der Drehkranz einen mit mindestens je einer Ein- und Ausströmöffnung für ein gasförmiges Kühlmedium versehenen abgedichteten Ringkanal begrenzen, in dem ein mit dem Drehkranz verbundenes Rohr liegt, das von einem Kühlmittel durchflossen ist, welches die Wärme von den elektrischen Verbrauchern auf den Drehkranz abführt.

Bei einem solchen in der EP-A-0 182 040 beschriebenen Computertomographen ist somit ein geschlossener Kühlkreislauf auf dem Drehkranz vorgesehen, in dem die Wärme von den Verbrauchern, insbesondere der Röntgenröhre, auf das Kühlmittel übertragen wird. Dieses durchströmt das Rohr im Ringkanal des Drehkranzes und gibt dort die Wärme an die als gasförmiges Kühlmedium verwendete Umgebungsluft ab, die den Ringkanal ausgehend von der Einströmöffnung in Richtung auf die Ausströmöffnung durchströmt. Dadurch erfolgt eine gezielte Wärmeabfuhr von dem Drehkranz nach außen. Nachteilig ist jedoch, daß dabei ein aus hygienischen Gründen unerwünschter Luftzug in dem Raum entsteht, in dem der Computertomograph aufgestellt ist. Es besteht zwar die Möglichkeit, einen solchen Luftzug zu vermeiden, indem die in dem Ringkanal strömende Luft durch entsprechende mit der Ein- und Ausströmöffnung verbundene Leitungen von außerhalb des Raumes zugeführt und nach außerhalb abgeführt wird, jedoch müssen dazu erhebliche bauliche Veränderungen an dem Raum vorgenommen werden. Außerdem sind die erwähnten Leitungen wegen ihres Raumbedarfs unerwünscht.

Auch bei dem aus der EP-A-0 109 206 bekannten Computertomographen wird eine ortsfeste Detektoranordnung mittels luft gekühlt, die aus dem Raum bezogen, bzw. an den Raum wieder abgegen wird.

Der Erfindung liegt die Aufgabe zugrunde, einen Computertomographen der eingangs genannten Art so auszubilden, daß auf einfache Weise ein Luftzug in dem Raum, in dem der Computertomograph aufgestellt ist, vermieden ist.

Nach der Erfindung wird diese Aufgabe dadurch gelöst, daß die Ausströmöffnung zur Bildung eines geschlossenen Kreislaufes für das in dem Ringkanal strömende Kühlmedium mit der Einströmöffnung verbunden ist, wobei zwischen beiden ein Wärmetauscher angeordnet ist, der außer von dem Kühlmedium von einem Kühlfluid beströmt ist. Da somit im Falle des erfindungsgemäßen Computertomographen ein geschlossener Kreislauf für das in dem Ringkanal strömende Kühlmedium vorhanden ist, kann ein aus hygienischen Gründen unerwünschter Luftzug nicht auftreten. Als Kühlfluid kommt entweder eine Flüssigkeit oder ein Gas in Frage. Im einfachsten Fall kann der Kühlkreislauf für das Kühlfluid offen sein und als Kühlfluid Leitungswasser Verwendung finden. Falls dies unerwünscht ist, ist nach einer Ausführungsform der Erfindung ein geschlossener Kühlkreislauf für das Kühlfluid vorgesehen, der ein Kühlaggregat zur Rückkühlung des Kühlfluids aufweist. Dabei ist das Kühlaggregat zweckmäßigerweise außerhalb des Raumes, in dem sich der Computertomograph befindet, angeordnet. Es wird dann nur ein äußerst geringer Teil der auf dem Drehkranz anfallenden Verlustwärme durch Strahlung in dem Raum, in dem sich der Computertomograph befindet, freigesetzt. Dies ist insbesondere dann von Vorteil, wenn der Raum, in dem sich der Computertomograph befindet, klimatisiert ist, da die Klimaanlage dann durch die von dem Computertomographen abgegebene Verlustwärme nicht nennenswert belastet wird.

Eine weitere Ausführungsform der Erfindung sieht vor, daß am Umfang des Ringkanales abwechselnd Ein- und Ausströmöffnungen vorgesehen sind und benachbarte Ein- und Ausströmöffnungen miteinander verbunden sind. Es wird so eine Steigerung der Kühlwirkung erzielt, da das durch den Ringkanal strömende Kühlmedium auf seinem Weg durch den Ringkanal mehrfach über Wärmetauscher geführt und rückgekühlt wird.

Eine besonders effektive Wärmeabfuhr erfolgt, wenn das Rohr im Ringkanal mehrere Windungen aufweist, weil in diesem Fall die wärmeabstrahlende Oberfläche im Ringkanal groß ist. Eine weitere Oberflächenvergrößerung ergibt sich, wenn die Außenseite des Rohres von einem Drahtgeflecht umgeben ist, das mit dem Rohr wärmeleitend verbunden ist. Im Gegensatz zu einer Verrippung des Rohres bietet das Drahtgeflecht den Vorteil, daß es den Strömungswiderstand, den das Rohr dem in dem Ringkanal strömenden Kühlmedium entgegensetzt, kaum erhöht. Im einfachsten Fall kann das Drahtgeflecht durch eine schraubenförmige Drahtwendel gebildet sein, die schraubenartig um das Rohr gewunden und an ihren Berührungspunkten mit dem Rohr stoffschlüssig, z.B. durch Löten, mit diesem verbunden ist.

Es ist möglich, auf dem Drehkranz einen Wärmetauscher vorzusehen, der einerseits die Wärme aus einem geschlossenen Kühlkreislauf für einen oder mehrere Verbraucher aufnimmt und andererseits an das Kühlmittel, das das Rohr im Ringkanal durchströmt, abgibt. Bei dieser Ausführung sind demgemäß zwei geschlossene Kühlkreisläufe auf dem Drehkranz und im Prinzip zwei Wärmetauscher vorhanden, nämlich einmal der Wärmetauscher auf dem Drehkranz und zum anderen das Rohr im Ringkanal, das ebenfalls einen Wärmetauscher darstellt. Diese Maßnahme bietet den Vorteil, daß bei einem Austausch von elektrischen Verbrauchern nur das in dem die Verbraucher umfassenden Kühlkreis befindliche Kühlmittel und nicht auch das in dem Rohr befindliche Kühlmittel entfernt werden muß. Als Kühlmittel für die geschlossenen Kühlkreisläufe kommt Öl in Frage. In dem Ringkanal kann Luft als Kühlmedium strömen.

Die Erfindung ist nachfolgend anhand eines in der Zeichnung dargestellten Ausführungsbeispieles näher erläutert. Es zeigen:

Fig. 1    die wesentlichen Teile eines Computertomographen nach der Erfindung,

Fig. 2    eine Einzelheit des Computertomographen gemäß Fig. 1, und

Fig. 3    eine weitere Einzelheit des Computertomographen gemäß Fig. 1.

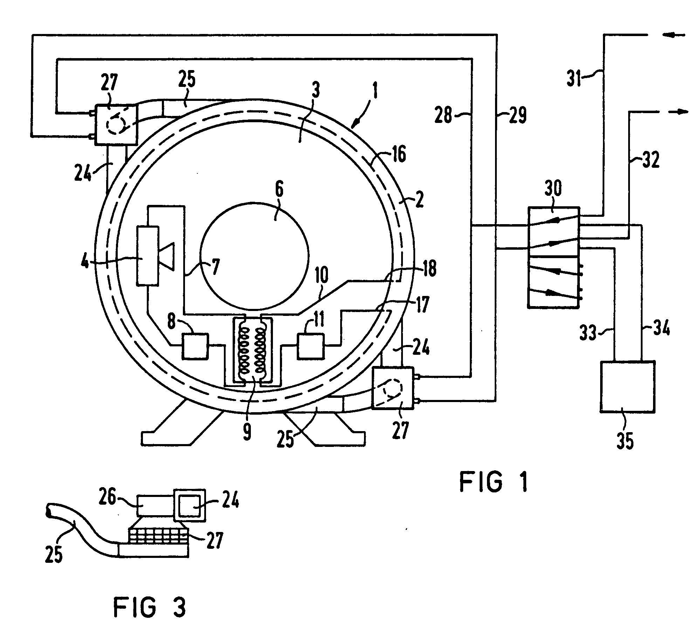

In der Fig. 1 ist ein Computertomograph 1 schematisch dargestellt, der einen feststehenden Geräteteil 2 und auf einem Drehkranz 3 einen Röntgenstrahler 4 und einen nicht dargestellten Strahlendetektor mit Meßelektronik aufweist. Zur Abtastung eines in einer Öffnung 6 liegenden Untersuchungsobjektes wird der Drehkranz 3 mit dem Röntgenstrahler 4 und dem Strahlendetektor um das Aufnahmeobjekt gedreht, so daß dieses aus einer Vielzahl von Richtungen durchstrahlt wird. Ein Rechner berechnet aus den jeweils empfangenen Strahlungsintensitäten die Schwächungswerte vorbestimmter Punkte in einer Schicht des Untersuchungsobjektes, die als Computertomogramm bildlich wiedergegeben werden können.

Zur Kühlung der auf dem Drehkranz 3 befindlichen elektrischen Verbraucher, in Fig. 1 ist von diesen nur der Röntgenstrahler 4 dargestellt, ist ein geschlossener Kühlkreislauf 7 mit einer Pumpe 8 und einem Wärmetauscher 9 vorgesehen. Das Kühlmittel in diesem Kühlkreislauf ist Öl. Dem Wärmetauscher 9 ist ein zweiter geschlossener Kühlkreislauf 10 mit einer Pumpe 11 zugeordnet, in dem ebenfalls Öl aus Kühlmittel strömt.

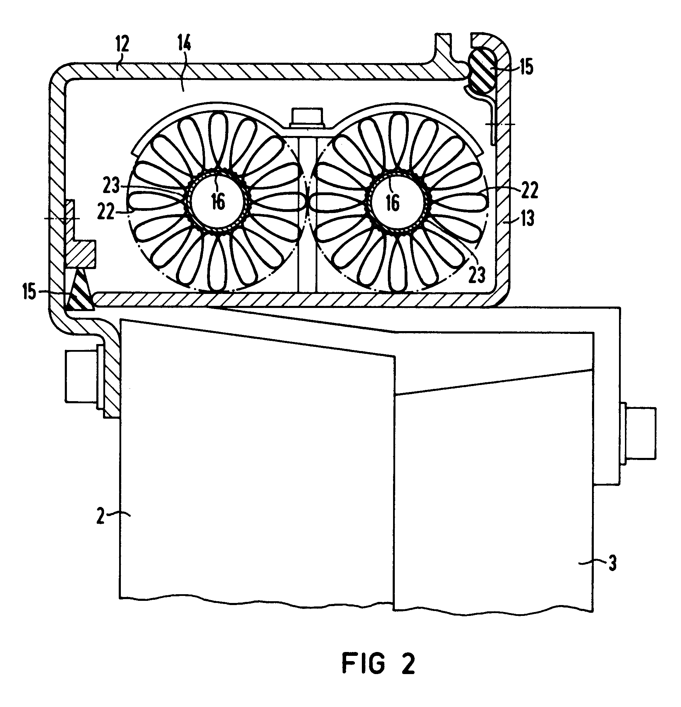

Aus der Fig. 2 geht hervor, daß der feststehende Geräteteil 2 des Computertomographen 1 und der Drehkranz 3 jeweils ein Gehäuseteil 12 bzw. 13 aufweisen, die zusammen einen Ringkanal 14 begrenzen, der durch Dichtungen 15 abgedichtet ist, welche eine Rotation des feststehenden Geräteteiles 2 und des Drehkranzes 3 bzw. der an diesen angebrachten Gehäuseteiles 12, 13 relativ zueinander erlauben. Im Ringkanal 14 liegen zwei im Schnitt dargestellte Windungen eines Rohres 16. Die beiden Enden des Rohres 16 sind gemäß Fig. 1 mit dem Einfluß 17 und dem Ausfluß 18 des Kühlkreislaufes 10 verbunden. Die im geschlossenen Gehäuse des Röntgenstrahlers 4 entstehende Wärme wird durch das das Gehäuse des Röntgenstrahlers 4 durchströmende Öl zum Wärmetauscher 9 geführt und dort auf das Kühlmittel des Kühlkreislaufes 10 übertragen. Im Ringkanal 14 wird die Wärme an ein diesen durchströmendes Kühlmedium übertragen, das bei dem Ausführungsbeispiel Luft ist.

Die Fig. 2 zeigt außerdem, daß das Rohr 16 auf seiner Außenseite die Oberfläche vergrößernde Mittel, nämlich ein Drahtgeflecht 22, aufweist. Das Drahtgeflecht 22 ist als schraubenförmige Drahtwendel ausgebildet, die schraubenartig um das Rohr 16 gewunden und an den Berührungspunkten 23 durch Löten wärmeleitend mit diesem verbunden ist. Durch diese Maßnahme wird die zur Wärmeabfuhr zur Verfügung stehende Oberfläche des Rohres 16 erheblich vergrößert.

Aus Fig. 1 ist ersichtlich, daß der Ringkanal 14 je zwei Einströmöffnungen 25 und Ausströmöffnungen 24 aufweist, die auf dessen Umfang abwechselnd angeordnet sind. Dabei sind je eine Ausströmöffnung 24 und eine Einströmöffnung 25 dicht beieinander angebracht und zur Bildung eines geschlossenen Kreislaufes für die Luft miteinander verbunden, wobei die in dem Ringkanal 14 strömende Luft, wie aus Fig. 3 ersichtlich, jeweils mittels eines Gebläses 26 über einen Wärmetauscher 27 geführt wird. Die Wärmetauscher 27 sind jeweils von einer Kühlflüssigkeit als Kühlfluid durchflossen, die über Leitungen 28 zu- und über Leitungen 29 abgeführt wird. Die Leitungen 28 und 29 sind jeweils zusammengeführt und führen zu einem Hahn 30, mittels dessen die Leitungen 28 und 29 wahlweise entweder zur Bildung eines offenen Kühlkreislaufes für die Kühlflüssigkeit mit einem Zulauf 31 und einem Ablauf 32 für die Kühlflüssigkeit, z.B. Leitungswasser, oder zur Bildung eines geschlossenen Kühlkreislaufes über die Leitungen 33 und 34 mit einem Kühlaggregat 35 verbunden werden können. Das Kühlaggregat 35 ist dabei zweckmäßigerweise außerhalb desjenigen Raumes angeordnet, in dem sich der Computertomograph befindet.

Es wird somit deutlich, daß im Falle des Ausführungsbeispieles die Wärmeabfuhr von den auf dem Drehkranz 3 befindlichen elektrischen Verbrauchern über wenigstens drei geschlossene Kühlkreisläufe erfolgt. Dies sind die Kühlkreisläufe 7, 10 und der Kreislauf der in dem Ringkanal 14 strömenden Luft. Infolge des Umstandes, daß die Luft in einem geschlossenen Kreislauf strömt, ist ein aus hygienischen Gründen unerwünschter Luftzug vermieden. Infolge der Kühlkreise 7 und 10 wird der Vorteil erzielt, daß bei einem Austausch von elektrischen Verbrauchern nur das in dem die Verbraucher umfassenden Kühlkreis 7 befindliche Kühlmittel und nicht auch das in dem Rohr 16 befindliche Kühlmittel des Kühlkreislaufes 10 entfernt werden muß.

**Patentansprüche**

1.  Computertomograph mit einem feststehenden Geräteteil (2), in dem ein Drehkranz (3) drehbar gelagert ist, der zu beiden Seiten einer den Patienten aufnehmenden Öffnung (6) einen Röntgenstrahler (4) und einen Strahlendetektor trägt, wobei der feststehende Geräteteil (2) und der Drehkranz (3) einen mit mindestens je einer Ein- und Ausströmöffnung (25 bzw. 24) für ein gasförmiges Kühlmedium versehenen abgedichteten Ringkanal (14) begrenzen, in dem ein mit dem Drehkranz (3) verbundenes Rohr (16) liegt, das von einem Kühlmittel durchflossen ist, welches die Wärme von elektrischen Verbrauchern (4) auf dem Drehkranz (3) abführt, **dadurch gekennzeichnet, daß** die Ausströmöffnung (24) zur Bildung eines geschlossenen Kreislaufes für das in dem Ringkanal (14) strömende Kühlmedium mit der Einströmöffnung (25) verbunden ist, wobei zwischen beiden ein Wärmetauscher (27) angeordnet ist, der außer von dem Kühlmedium von einem Kühlfluid beströmt ist.

2.  Computertomograph nach Anspruch 1, **dadurch gekennzeichnet, daß** ein geschlossener Kühlkreislauf für das Kühlfluid vorgesehen ist, der ein Kühlaggregat (35) zur Rückkühlung des Kühlfluids aufweist.

3.  Computertomograph nach Anspruch 1 oder 2, **dadurch gekennzeichnet, daß** am Umfang des Ringkanales (14) abwechselnd Ein- und Ausströmöffnungen (25) bzw. (24) vorgesehen sind und benachbarte Ein- und Ausströmöffnungen (25 bzw. 24) miteinander verbunden sind.

4.  Computertomograph nach einem der Ansprüche 1 bis 3, **dadurch gekennzeichnet, daß** das Rohr (16) im Ringkanal (14) mehrere Windungen aufweist.

5.  Computertomograph nach einem der Ansprüche 1 bis 4, **dadurch gekennzeichnet, daß** auf der Außenseite des Rohres (16) die Oberfläche vergrößernde Mittel (22) angeordnet sind.

6.  Computertomograph nach Anspruch 5, **dadurch gekennzeichnet, daß** die Außenseite des Rohres (16) von einem Drahtgeflecht (22) umgeben ist, das mit dem Rohr (16) wärmeleitend verbunden ist.

7.  Computertomograph nach Anspruch 6, **dadurch gekennzeichnet, daß** das Drahtgeflecht (22) durch eine schraubenförmige Drahtwendel gebildet ist, die schraubenartig um das Rohr (16) gewunden ist.

8.  Computertomograph nach einem der Ansprüche 1 bis 7, **dadurch gekennzeichnet, daß** auf dem Drehkranz (3) ein Wärmetauscher (9) vorgesehen ist, der einerseits die Wärme aus einem geschlossenen Kühlkreislauf (7) für die elektrischen Verbraucher (4) aufnimmt und andererseits an das Kühlmittel, das das Rohr (16) im Ringkanal (14) durchströmt, abgibt.

**Claims**

1.  Computerised tomography apparatus, having a stationary part (2) in which there is rotatably mounted a rotating ring (3) which carries an X-ray source (4) and a radiation detector on either side of an opening (6) which receives the patient, the stationary part (2) and the rotating ring (3) bounding a sealed annular channel (14) provided with at least one inlet and one outlet opening (25 and 24 respectively) for a gaseous cooling medium, in which sealed annular channel (14) there is located a tube (16) connected to the rotating ring (3), through which tube (16) there flows a coolant which carries away the heat from electrical loads (4) on the rotating ring (3), **characterised in that** the outlet opening (24), in order to form a closed circuit for the cooling medium flowing in the annular channel (14), is connected to the inlet opening (25), with there being arranged between these two openings a heat exchanger (27) which has, besides the cooling medium, a cooling fluid flowing through it.

2.  Computerized tomography apparatus according to claim 1, **characterised in that** a closed cooling circuit is provided for the cooling fluid, which cooling circuit has a cooling unit (35) for the recooling of the cooling fluid.

3.  Computerised tomography apparatus according to claim 1 or 2, **characterised in that,** on the periphery of the annular channel (14), there are provided alternately inlet and outlet openings (25) and (24) respectively, and adjacent inlet and outlet openings (25 and 24 respectively) are connected together.

4.  Computerised tomography apparatus according to one of claims 1 to 3, **characterised in that** the tube (16) in the annular channel (14) has several turns.

5. Computerised tomography apparatus according to one of claims 1 to 4, **characterised in that,** on the outside of the tube (16), there are arranged means (22) for enlarging the surface area.

6. Computerised tomography apparatus according to claim 5, **characterised in that** the outside of the tube (16) is surrounded by a wire mesh (22) which is connected in heat-conducting manner to the tube (16).

7. Computerised tomography apparatus according to claim 6, **characterised in that** the wire mesh (22) is formed by a helical wire filament which is wound in helical manner around the tube (16).

8. Computerised tomography apparatus according to one of claims 1 to 7, **characterised in that,** on the rotating ring (3), there is provided a heat exchanger (9) which, on the one hand, absorbs the heat from a closed cooling circuit (7) for the electrical loads (4) and, on the other hand, transfers this heat to the coolant which flows through the tube (16) in the annular channel (14).

**Revendications**

1. Tomodensitomètre comportant une partie d'appareil fixe (2), dans laquelle est montée de façon à pouvoir tourner une couronne rotative (3), qui porte, des deux côtés d'une ouverture (6) logeant le patient, un émetteur (4) de rayons X et un détecteur de rayonnement, et dans lequel la partie d'appareil fixe (2) et la couronne rotative (3) limitent un canal annulaire étanchéifié (14), qui comporte au moins respectivement une ouverture d'entrée et une ouverture de sortie (25 et 24) pour un milieu de refroidissement gazeux et dans lequel est disposé un tube (16), qui est raccordé à la couronne rotative (3) et est traversé par un milieu de refroidissement qui évacue la chaleur d'appareils d'utilisation électriques (4) situés sur la couronne rotative (3), caractérisé par le fait que l'ouverture de sortie (24) est raccordée à l'ouverture d'entrée (25) pour former un circuit fermé pour le fluide de refroidissement circulant dans le canal annulaire (14), tandis qu'entre ces ouvertures est disposé un échangeur de chaleur (27) qui est traversé non seulement par le milieu de refroidissement, mais également par un fluide de refroidissement.

2. Tomodensitomètre suivant la revendication 1, caractérisé par le fait qu'il est prévu un circuit fermé de refroidissement pour le fluide de refroidissement, qui comprend un agrégat de refroidissement (35) pour réaliser le refroidissement au retour du fluide de refroidissement.

3. Tomodensitomètre suivant la revendication 1 ou 2, caractérisé par le fait que des ouvertures d'entrée et de sortie (25) et (24) sont prévues alternativement sur le pourtour du canal annulaire (14) et que des ouvertures d'entrée et de sortie voisines (25 et 24) sont raccordées entre elles.

4. Tomodensitomètre suivant l'une des revendications 1 à 3, caractérisé par le fait que le tube (16) situé dans le canal annulaire (14) comporte plusieurs spires.

5. Tomodensitomètre suivant l'une des revendications 1 à 4, caractérisé par le fait que des moyens (22), qui augmentent la surface, sont disposés sur la face extérieure du tube (16).

6. Tomodensitomètre suivant la revendication 5, caractérisé par le fait que la face extérieure du tube (16) est entourée par une tresse de fils (22) qui est raccordée de façon thermoconductrice au tube (16).

7. Tomodensitomètre suivant la revendication 6, caractérisé par le fait que la tresse de fils (22) est formée par un filament hélicoïdal enroulé selon une disposition hélicoïdale autour du tube (16).

8. Tomodensitomètre suivant l'une des revendications 1 à 7, caractérisé par le fait que sur la couronne rotative (30), il est prévu un échangeur de chaleur (9) qui, d'une part, absorbe la chaleur délivrée par un circuit fermé de refroidissement (7) pour les appareils d'utilisation électriques (4), et, d'autre part, délivre la chaleur au milieu de refroidissement, qui traverse le tube (16) situé dans le canal annulaire (14).

**FIG 1**

**FIG 3**

FIG 2